# EUROPEAN PATENT SPECIFICATION

(11) **EP 2 276 453 B1**
(45) Date of publication and mention of the grant of the patent: **27.08.2014**
(21) Application number: 08763800.3
(22) Date of filing: 11.04.2008
(51) Int. Cl.: A61K 8/44, A61Q 19/10

(54) **Solubilizing agent for essential oils and cosmetic formulations containing them**
Solubilisierendes Mittel für essentielle Öle und sie enthaltende kosmetische Zubereitungen
Agent solubilisant pour huiles essentielles et compositions cosmétiques les contenant

(43) Date of publication of application: 26.01.2011
(73) Proprietor: Maycos Italiana S.A.S., 46043 Castiglione delle Stiviere (MN) (IT); Sinerga Group S.r.l., 20154 Milano (IT)
(72) Inventor: COMINI, Palmiro, I-46043 Castiglione Delle Stiviere (IT); TERZI, Gianluigi, I-46043 Castiglione Delle Stiviere (IT); LENZINI, Marina, deceased (IT)
(74) Representative: Palladino, Saverio Massimo
(86) International application number: PCT/IT2008/000237
(87) International publication number: WO 2009/125441

(56) References cited:
- EP-A- 1 444 973
- WO-A-2007/003658
- WO-A-2008/061990
- FR-A- 2 340 981
- FR-A- 2 892 920
- FR-A1- 2 892 020
- JP-A- 1 023 000
- US-B1- 6 296 859
- ORGANAT RAW MATERIAL FOR COSMETIC INDUSTRY, [Online] XP002511298 Retrieved from the Internet: URL:http://www.organat.com/index.php?optio n=com_content&task=view&id=154&Itemid=124> [retrieved on 2009-01-21]

## Description

### Field of the invention

The present invention relates to a solubilizing agent for essential oils to be used mainly in essentially aqueous or aqueous-alcoholic cosmetic and pharmaceutical formulations such as perfume, eau de cologne, tonic lotion, and in water-based cleansing formulations such as bath foam, shower foam, shampoo and liquid soap.

The present invention relates to a pre-formulated product containing said solubilizing agent in combination with said essential oil suitable for preparing the aforesaid aqueous cosmetic formulations and cleansing compositions.

Finally a further aspect of the present invention are the aforesaid aqueous formulations for cosmetic use and cleansing compositions.

### State of the art

In the cosmetics industry, non-ionic surfactants derived from ethylene oxide are required to water-solubilize essential oils and fragrances, whether of natural or synthetic origin, and to obtain clear solutions. It is now widely known that these substances are damaging to the skin, as metabolites of the oxyethylene chain are toxic. Moreover, these types of substance are non-biodegradable and therefore highly polluting. FR-A-2390981 discloses the use of ethoxylated nonionic surfactants for solubilizing essential oils.

Today's cosmetics and cleansing products industries aim to use substances which give the same level of performance while at the same time are non-toxic to the skin and have a low environmental impact.

Lipo-amino acids, or rather N-acyl amino acid derivatives in which the acyl group is derived from a fatty acid, are already widely known as their use in the cosmetics industry is also widely known.

For example, FR2771632 describes a composition containing a lipo-amino acid and glycol combination with antimicrobial activity for the treatment of acne; EP1147765 A1 describes a cosmetic composition containing as active ingredients a lipo-amino acid and a salt of methicone copolyol phosphate with skin protecting action. In EP1013266, lipo-amino acids are used in cosmetic formulations as texture agents, or as coatings for cosmetic powders.

Lipo-amino acids are also used as surfactants in oil-based cosmetic cleansing compositions as described in WO2005/013927.

Finally WO2007/003658, in the name of the applicant, describes a lipogel containing at least one lipophilic substance, at lease one lipoprotein and at least one lipo-amino acid, again for use in the cosmetics and cleansing fields.

Some of the lipo-amino acids cited in the aforesaid prior art, such as glutamate and glycine derivatives, are barely effective as solubilizers of oily components in water.

### Summary of the invention

The applicant has now surprisingly found a solubilizing agent for essential oils in water-based compositions comprising at least one N-acyl derivative of proline, of formula (I) 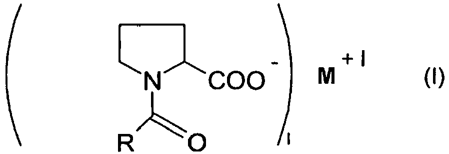 in which R is an alkyl radical from 5 to 21 carbon atoms, and is obtained from fatty acids contained in coconut oil, M is chosen from sodium and potassium, I is 1, wherein the weight ratio between the solubilizing agent and the essential oil is comprised between 0.1:1 and 10:1.

The applicant has also found that the addition of a salt formed from an organic or inorganic monovalent cation and the corresponding anion of an organic or inorganic acid substantially enhances (in some cases by 1 or 2 fold) the solubilizing activity of the solubilizer of formula (I).

A further aspect of the present invention is therefore a pre-formulated product for water-based compositions, containing essential oil and the solubilizer of formula (I) possibly in association with the aforesaid salt.

A further aspect of the present invention are essentially aqueous or aqueous-alcoholic cosmetic formulations, and water-based cleansing formulations containing essential oil and the solubilizer of formula (I) optionally added with said salt.

### Description of the figures

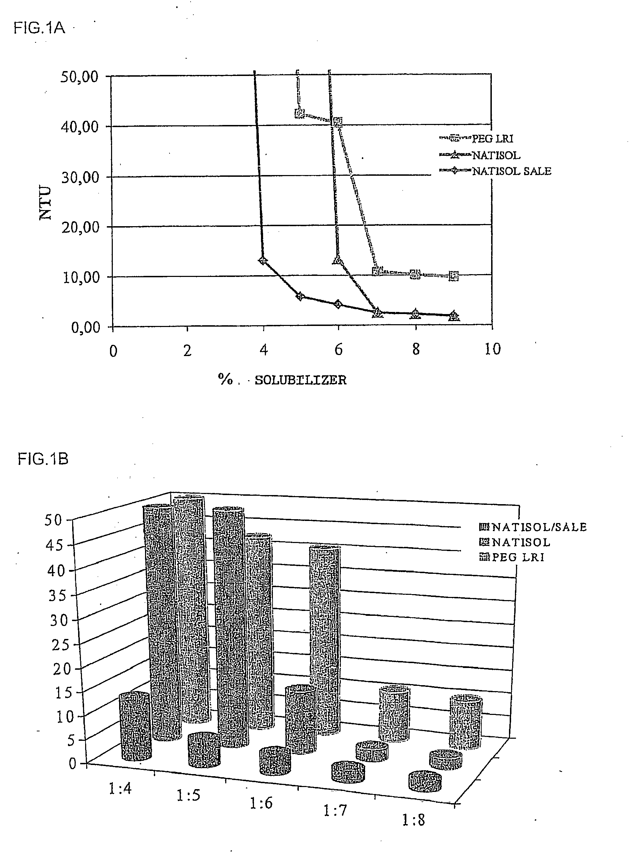

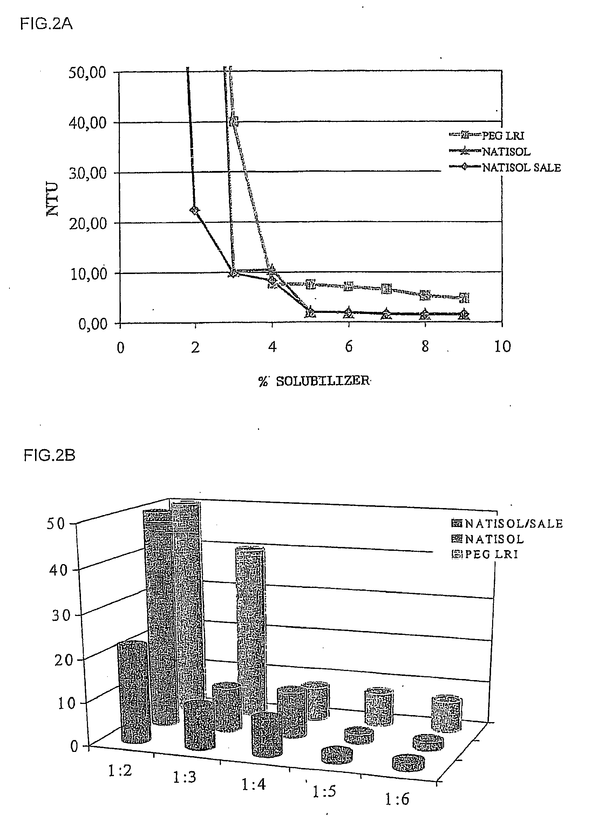

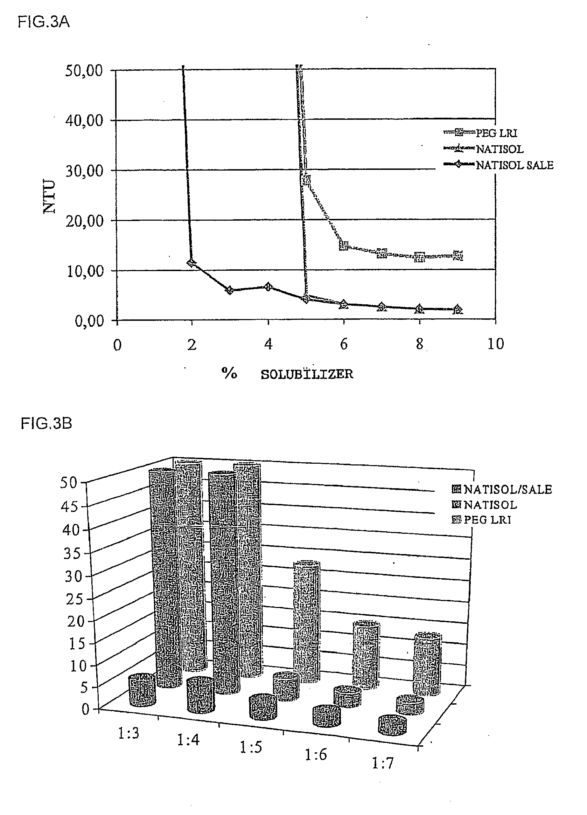

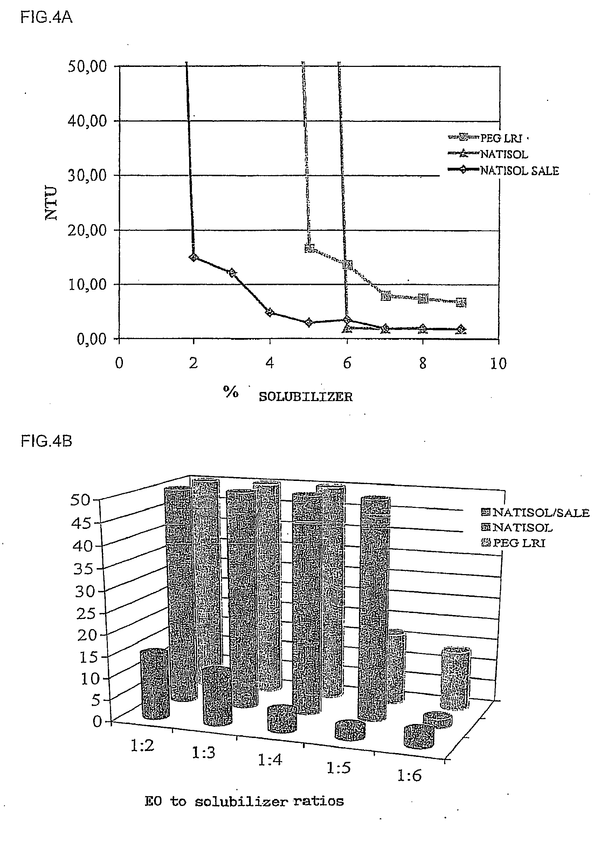

Figures 1 A, 2A, 3A, 4A are graphs showing the solubilizing capacity of cocoyl proline sodium salt (NATISOL) and cocoyl proline sodium salt added with sodium chloride (NATISOL/SALT) compared to that of the known surfactant (PEG LRI), in aqueous formulations containing respectively: 1% essential oil of basil (Fig. 1A), eucalyptus (Fig. 2A), mint (Fig. 3A) and sage (Fig. 4A), where the y-axis shows the nephelometric turbidity units (NTU) and the x-axis shows the % of solubilizing oil.
Figures 1B, 2B, 3B 4B are three-dimensional graphs showing the solubilizing capacities of a known surfactant (PEG LRI), cocoyl proline sodium salt (NATISOL) and cocoyl proline sodium salt with added sodium chloride (NATISOL/SALT) relating to the respective aqueous formulations of Fig. 1A-4A, as a function of the essential oil:solubilizing agent ratio.

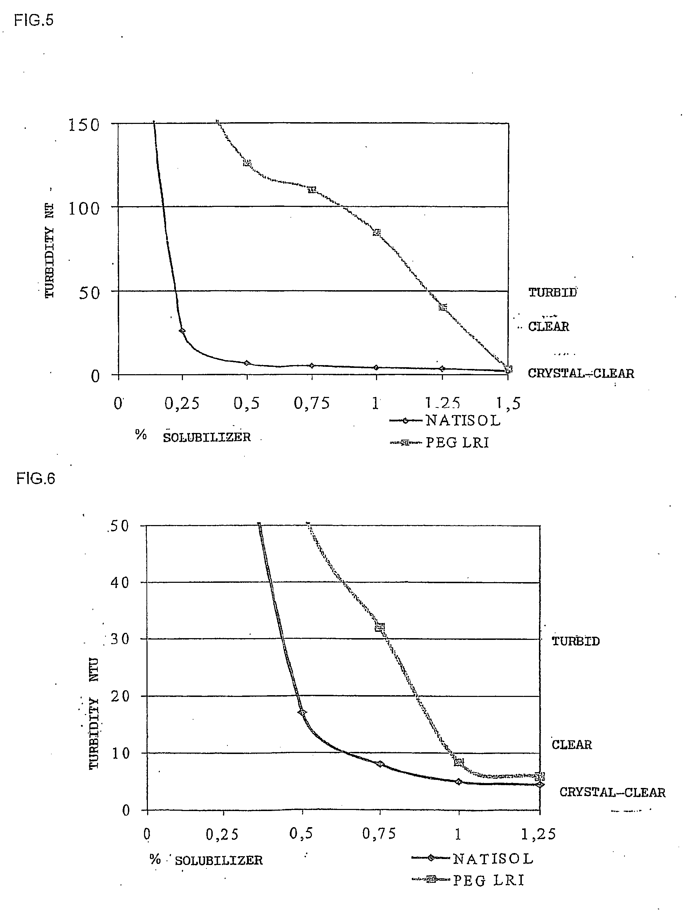

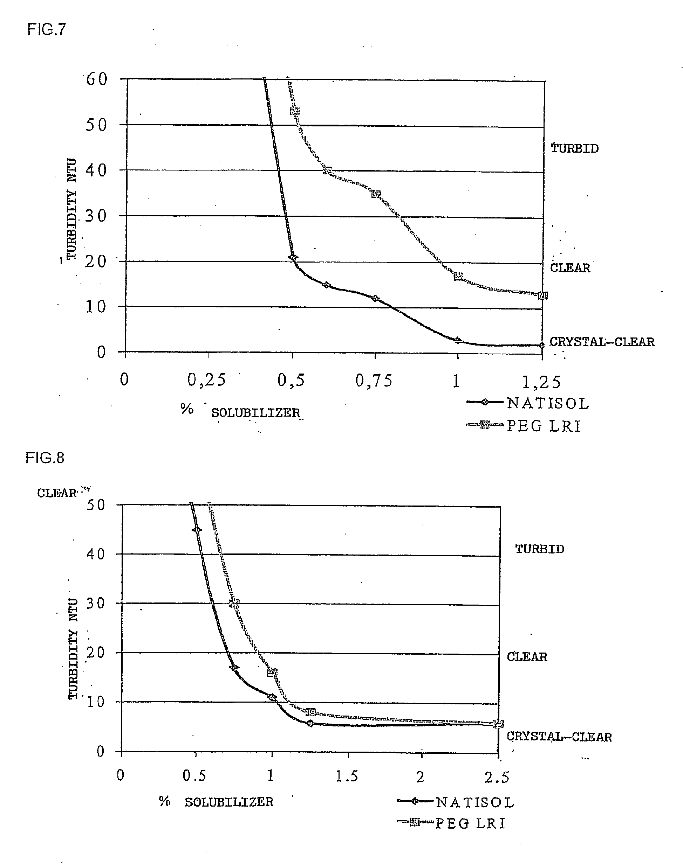

Figures 5, 6, 7, 8 are graphs showing the solubilizing capacities of cocoyl proline sodium salt (NATISOL) and a known surfactant (PEG LRI) for bath foams, containing respectively: 1 % essential oil of pine (Fig. 5), lavender (Fig. 6), basil (Fig. 7.), lemon (Fig. 8). Specifically, the x-axis shows the solubilizing agent concentrations in the aqueous formulations, while the y-axis shows turbidity units (NTU).

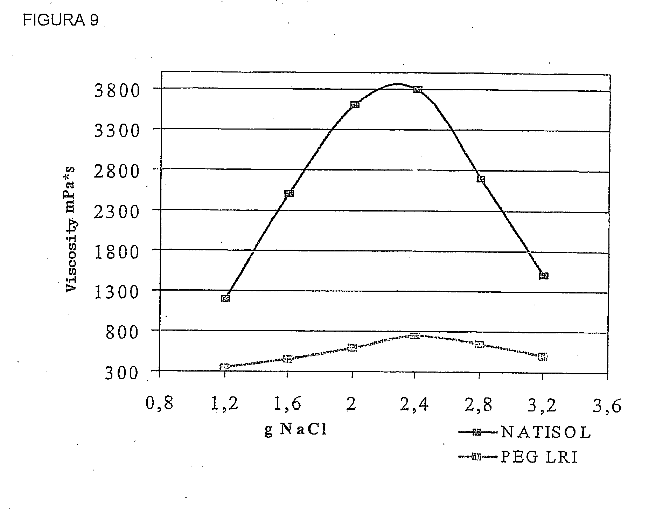

Finally, figure 9 shows the variation in viscosity of bath foam containing 1 % mint essential oil with added known solubilizer (PEG LRI) at 3% and cocoyl proline sodium salt (NATISOL), as a function of added sodium chloride.

### Detailed description of the invention

For the purposes of the present invention water-based compositions include essentially aqueous and aqueous-alcoholic cosmetic formulations such as perfume, cologne, after-shave, lotions, tonics, as well as cleansing formulations of different viscosities, such as bath foam, shampoo, shower foam and liquid soap. Preferably the solubilizer of the present invention consists of a mixture of the N-acyl derivates of formula (I) having different, preferably saturated, alkyl residues, and having preferably from 7 to 20 carbon atoms.

Said mixtures are N-acyl derivatives of proline with fatty acids contained in coconut oils.

The solubilizer consists of a mixture of N-acyl derivatives of proline with coconut oil fatty acids, which will hereinafter be defined as cocoyl proline. In the compounds of formula (I) M is chosen sodium and potassium,

The solubilizing agent of formula (I) is cocoyl proline sodium or potassium salt obtained by condensing proline with coconut fatty acids. Preferably this solubilizing agent is mixed with water at a concentration between 30 and 70% by weight, being even more preferably between 55 and 65%. In this case it is a clear viscous liquid of pH 6.0-7.5 with a light straw colour and a density of 1.08-1.12 g/ml.

The pre-formulated products for water-based compositions, being a further aspect of the present invention, contain essential oil and the solubilizing agent of formula (I); the latter is added to the essential oil in weight ratios, relative to the essential oil, dependent on the specific essential oil and are comprised between 0.1:1 and 10:1.

If said pre-formulated product is intended for the preparation of essentially aqueous or aqueous-alcoholic cosmetic formulations, such as perfume, eau de cologne, after shave, lotions and tonics, said ratio is preferably comprised between 3:1 and 8:1.

In this case if the relative aqueous formulations contain 1% essential oil, they will contain between 3 and 8% cocoyl proline sodium or potassium salt.

If the pre-formulated product is intended instead for the preparation of water-based cleansing formulations, the weight ratios of cocoyl proline sodium or potassium salt to essential oil are preferably comprised between 0.2:1 and 5:1. In this case if the final cleansing compositions contain 1% essential oil, they will contain between 0.2 and 5% by weight of solubilizer.

Preferably the pre-formulated product also contains salt formed from an inorganic monovalent cation such as an alkali metal, ammonium, or organic cation such as an organic amine cation, and an anion of an organic or inorganic acid in addition to the solubilizing agent of the present invention.

Preferably this salt is chosen from sodium chloride, sodium lactate, ammonium chloride, and is even more preferably chosen from sodium chloride and sodium lactate.

If the solubilising agent of the present invention is cocoyl proline sodium or potassium salt and the salt is sodium chloride or sodium lactate, the weight ratio of solubilizer:salt:essential oil/fragrance is preferably comprised between 0.1:10:1 and 10:0.1:1.

If the pre-formulated product is specifically used for preparing essentially aqueous or aqueous-alcoholic cosmetic formulations, the weight ratio of cocoyl proline sodium or potassium salt:sodium lactate or sodium chloride:essential oil and/or fragrance is preferably comprised between 1:10:1 and 6:0.1:1.

Hence in this case, if the essentially aqueous or aqueous-alcoholic cosmetic formulations of the present invention contain 1 % essential oil, they will therefore contain between 1 and 6% by weight of cocoyl proline sodium or potassium salt and between 0.1 and 10% by weight of sodium chloride or sodium lactate.

If the pre-formulated product is used for the preparation of water-based cleansing compositions and the solubilizing agent of the present invention is cocoyl proline sodium or potassium salt and the salt is sodium chloride or sodium lactate, the weight ratio of cocoyl proline sodium or potassium salt:sodium chloride:essential oil is preferably comprised between 0.25:4:1 and 5:0.5:1.

In this case if the cleansing compositions obtained using these pre-formulated products contain 1% essential oil, they will contain between 0.25 and 5% by weight of cocoyl proline sodium or potassium salt and between 0.5 and 4% by weight of sodium chloride or sodium lactate.

The following examples of the preparation of the essentially aqueous cosmetic formulations and of the water-based cleansing formulations containing various types of essential oils solubilized with cocoyl proline sodium salt are provided by way of non-limiting illustration, the degree of solubility of said formulations being compared with commercially known surfactants.

### Examples

The degree of solubility of said water-based compositions was assessed on the basis of their clarity, determined by turbidimetric measurements with a portable nephelometric TB1 turbidimeter by Velp Scientifica.

The nephelometric turbidimeter is based on a comparison between the intensity of a light beam scattered through a sample and the intensity of a light beam scattered through a reference standard under the same sample conditions. The electronic light detector is positioned at 90° to the light source and measures reflected light, which strikes the surface of the sample tube. The greater the light reflected, the greater the sample turbidity.

Solutions are considered to be clear if the turbidity value (NTU) is less than 30. It can be deduced that the lower this value, the clearer the solution. Below a value of 5 NTU, the clarity is said to be crystal-clear.

### Example 1: Essentially aqueous formulations

### Preparation

The essential oil, the cocoyl proline sodium salt and the optional sodium chloride added in the form of a 20% aqueous solution are mixed with a magnetic stirrer in the ratios indicated in the following tables, adding sufficient water so that the aqueous formulation weighs 100 g in total.

The solubilizing capacity of the cocoyl proline sodium salt optionally combined with sodium chloride was compared with a solubilizing agent having the following composition:
◆ 40-50% PEG-40 Hydrogenated castor oil
◆ 30-40% PPG-26 Buteth-26
◆ 10-15% Trideceth-9
◆ 5-10% Water

For the sake of simplicity, in the subsequent pages this type of product will be known as "PEG LRI".

In the following tables the degrees of solubility of the essentially aqueous formulations obtained with the solubilizing agent of the present invention and optional added sodium chloride are given and compared with those obtained with the state of the art surfactant.

**Table 1. Aqueous formulation containing basil essential oil**

| Essential oil: Solubilizer (g/g) | Clarity (NTU) PEG LRI | Clarity (NTU) Cocoyl proline sodium salt | Clarity (NTU) Cocoyl proline sodium salt + sodium chloride | Sodium chloride (g) |
|---|---|---|---|---|
| 1:1 | 1000 | 1000 | 1000 | - |
| 1:2 | 1000 | 1000 | 1000 | - |
| 1:3 | 986 | 1000 | 1000 | - |
| 1:4 | 525 | 920 | 13.10 | 2.5 |
| 1:5 | 42.3 | 840 | 5.81 | 1.4 |
| 1:6 | 40.5 | 13.29 | 4.20 | 0.7 |
| 1:7 | 10.70 | 2.56 | 2.56 | - |
| 1:8 | 10.05 | 2.30 | 2.30 | - |
| 1:9 | 9.69 | 1.90 | 1.90 | - |

The data given in table 1 have also been represented in the form of a two-dimensional and a three-dimensional graph.

As apparent from both the tabulated data and graphs in Figures 1A and 1B, the solution clarifies when using respectively a 7:1 ratio of known solubilizer:essential oil (7% solubilizer in the final aqueous formulation) but only 6:1 (6% of solubilizer) with the solubilizer of the invention; the solubilizer:essential oil ratio is further reduced to 4:1 (4% of solubilizer in the final aqueous formulation) if sodium chloride (2.5 g) is also added to the solubilizer of the present invention.

Neither does the aqueous formulation become crystal-clear by using a high solubilizer:essential oil ratio of 9:1 with the solubilizer of the known art, whereas with the solubilizer of the present invention it already becomes crystal-clear by using only a 7:1 solubilizer:essential oil ratio (7% in the final formulation); this ratio falls further to 6:1 by adding only 0.7 g of sodium chloride.

**Table 2. Formulation containing eucalyptus essential oil**

| Essential oil: Solubilizer (g/g) | Clarity (NTU) PEG LRI | Clarity (NTU) Cocoyl proline sodium salt | Clarity (NTU) Cocoyl proline sodium salt + sodium chloride (g) | Sodium chloride (g) |
|---|---|---|---|---|
| 1:1 | 1000 | 1000 | 1000 | - |
| 1:2 | 787 | 741 | 22.43 | 5.4 |
| 1:3 | 40.0 | 10.17 | 10.5 | 1.2 |
| 1:4 | 7.71 | 10.54 | 11.59 | 0.85 |
| 1:5 | 7.56 | 2.08 | 7.60 | 0.40 |
| 1:6 | 7.04 | 1.90 | 2.87 | 0.4 |
| 1:7 | 6.59 | 1.71 | 1.71 | - |
| 1:8 | 5.29 | 1.60 | 1.60 | - |
| 1:9 | 4.74 | 1.60 | 1.60 | - |

The data given in table 2 have also been represented in the form of a two-dimensional and a three-dimensional graph in Figures 2A and 2B respectively.

As apparent from both the tabulated data and graphs in Figures 2A and 2B, the solution clarifies when using a 4:1 ratio of known solubilizer:essential oil (4% in the final aqueous formulation), whereas with the cocoyl proline sodium salt, in accordance with the present invention the solution clarifies when using a solubilizing agent:essential oil ratio of 3:1 (3% of solubilizer in the final aqueous formulation); if sodium chloride (5.4 g) is also added, this ratio is further reduced to 2:1.

The aqueous formulation becomes clear when using a high known-art solubilizer:essential oil ratio of 9:1 (9% of solubilizer in the final aqueous formulation), whereas this ratio is lower at 5:1 when using cocoyl proline sodium salt (5% in the final aqueous formulation). Addition of sodium chloride does not affect the solubilizing capacity of the cocoyl proline sodium salt.

**Table 3. Formulation containing mint essential oil**

| Essential oil: solubilizer (g/g) | Clarity (NTU) PEG LRI | Clarity (NTU) Cocoyl proline sodium salt (60%) | Clarity (NTU) Cocoyl proline sodium salt + sodium chloride | Sodium chloride (g) |
|---|---|---|---|---|
| 1:1 | 1000 | 1000 | 256 | 9.7 |
| 1:2 | 1000 | 1000 | 11.52 | 5g |
| 1:3 | 1000 | 890 | 5.96 | 1.4 |
| 1:4 | 976 | 863 | 6.61 | 0.8 |
| 1:5 | 27.9 | 4.96 | 4.10 | 0.3 |
| 1:6 | 14.76 | 3.10 | 3.10 | - |
| 1:7 | 13.20 | 2.60 | 2.60 | - |
| 1:8 | 12.41 | 2.10 | 2.10 | - |
| 1:9 | 12.50 | 2.05 | 2.05 | - |

The data given in table 3 have also been represented in the form of a two-dimensional and a three-dimensional graph in Figures 3A and 3B respectively. As apparent from both the tabulated data and graphs in Figures 3A and 3B, the solution clarifies when using respectively a 5:1 ratio of known solubilizer:essential oil (5% in the final aqueous formulation) whereas with the cocoyl proline sodium salt and using the same ratio, the formulation actually becomes crystal-clear. A clear solution is instead obtained using a low cocoyl proline sodium salt:essential oil ratio of 2:1 (2% in the final solution) if 5 g of sodium chloride are added.

**Table 4_{.} Formulation containing sage essential oil**

| Essential oil: solubilizer (g/g) | Clarity (NTU) PEG LRI | Clarity (NTU) Cocoyl proline sodium salt | Clarity (NTU) Cocoyl proline sodium salt + sodium chloride | Sodium chloride (g) |
|---|---|---|---|---|
| 1:1 | 1000 | 1000 | 1000 | - |
| 1:2 | 1000 | 1000 | 15.03 | 9.1 |
| 1:3 | 1000 | 1000 | 12.16 | 3 |
| 1:4 | 505 | 564 | 4.77 | 2.1 |
| 1:5 | 16.69 | 443 | 2.90 | 1.05 |
| 1:6 | 13.53 | 2.02 | 3.44 | 0.2g |
| 1:7 | 7.87 | 1.90 | 1.90 | - |
| 1:8 | 7.39 | 1.90 | 1.90 | - |
| 1:9 | 6.72 | 1.85 | 1.85 | - |

The data given in table 4 have also been represented in the form of a two-dimensional and a three-dimensional graph in Figures 4A and 4B respectively.

As apparent from both the tabulated data and graphs in Figures 4A and 4B, the solution clarifies when using a 5:1 ratio of known solubilizer:essential oil (5% in the final aqueous formulation) whereas with the cocoyl proline sodium salt, a ratio of only 6:1 need be used for the solution to be already crystal-clear. This ratio drops to 4:1 when 2.1 g of sodium chloride are added.

In conclusion in the essential oils tested, cocoyl proline sodium salt has a comparable solubilizing capacity to that of the known surfactant, although in the illustrated cases it is actually higher. However, if moderate amounts of only 1-5% sodium chloride are added to the cocoyl proline sodium salt, the latter shows a solubilizing capacity 1 or 2 times greater than that of the known surfactant.

### Example 2: Water-based cleansing formulations

The solubilizing agents of the present invention are an important option for formulating skin-compatible and ecological cosmetic products (containing essential oils to be solubilized).

As already shown for these products, solubilization of the various essential oils in cosmetic cleansers can be enhanced, particularly in products containing high amount of essential oils such as bath foam and shower foam which often have clarity problems and hence require the aid of ethoxylated substances.

For example a bath foam with 1% essential oil remains turbid. If this EO (essential oil) is first dissolved in a 1:0.5 ratio (EO:cocoyl proline sodium salt), it spontaneously clarifies in the bath foam.

It can be stated that cocoyl proline sodium salt is a potent solubilizer of vegetable origin for cosmetic cleansers, being also active at low concentrations.

By way of illustration, figures 5-8 show some non-limiting examples of the solubilizing capacity of cocoyl proline sodium salt compared with PEG LRI whose composition is given above, in a standard cleansing formula composed as follows:
30% SLES (28% sodium lauryl ether sulphate)
3% of 30% Cocobetaine
1% Cocamide DEA (diethanolamine)
1% Essential oil and to 100% with water

Figure 5 shows a graph of variation in the solubility of bath foam containing pine essential oil from which it can be seen that a concentration of just 0.25% of the solubilizer of the present invention is sufficient to transform the bath foam from turbid to clear, while with the known surfactant a quantity of 1.25%, that is to say a quantity equal to 5 times the quantity of cocoyl proline sodium salt, is necessary.

Figure 6 clearly shows that if about 0.7% by weight of the solubilizer of the present invention is added to bath foam containing lavender oil, a clear bath foam is obtained whereas about 1% of the known surfactant must be added to attain comparable results.

Figure 7 demonstrates that if about 0.5% of the solubilizer of the present invention is added to a bath foam containing basil oil, a clear bath foam can be obtained; with the known surfactant, a quantity of 1.25% is required to attain comparable results.

Figure 8 demonstrates that in a bath foam containing lemon oil the solubilizer of the present invention is more effective than the known product even though to a lesser extent than in the other aforegiven examples.

We can therefore state that if the solubilizer of the present invention is introduced into a surfactant system such as cosmetic cleansers, it is far more effective than traditional solubilizers for solubilizing significant quantities of essential oil, in some cases by up to 100-200%, and with considerable savings. Moreover, it does not reduce viscosity as do oxyethylene derivatives but results in a higher decreasing viscosity curve as indicated in the graph shown in figure 9.

In this respect if we examine a basic cleansing formula composed as follows:
30% of 28% SLES
3% of 30% Cocobetaine
1 % Cocamide DEA
1% mint essential oil
% NaCl as shown in the x-axis in figure 8
3% solubilizer (cocoyl proline/PEG-LRI)
to 100% with water,
it can be noted that bath foam viscosity does not decrease and is much higher than that of the bath foam containing the known solubilizer.

Another point greatly in favour of cocoyl proline sodium salt usage is the nature of said compound, being a lipo-amino acid very gentle on the skin, as it is derived from one of the amino acids with the most presence thereon, i.e. proline. Furthermore, it has a strong foam-producing capacity and, in contrast to oxyethylated castor oil solubilizers, does not knock down foam but strengthens it significantly such as to replace any secondary surfactants suited to this purpose. The solubilizers of the present invention, being foam-producing lipo-amino acids, also become secondary surfactants with positive effects on skin hydration; below pH 5, however, they lose their activity in systems devoid of surfactants and must hence, if required, be supported by traditional solubilizers.

## Claims

1. Aqueous composition containing an essential oil and a solubilizing agent for said essential oil, said solubilizing agent comprising at least one N-acyl derivative of proline of formula (I) 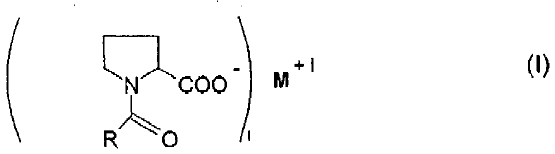 in which R is an alkyl radical from 5 to 21 carbon atoms and is obtained from fatty acids contained in coconut oil, M is chosen from sodium or potassium, I is 1, wherein the weight ratio between the solubilizing agent and the essential oil is comprised between 0.1:1 and 10:1.

2. Aqueous composition according to claim 1, wherein the solubilizing agent consists of a mixture of N-acyl derivatives of formula (I) having different saturated alkyl residues R having from 7 to 20 carbon atoms.

3. Aqueous or aqueous-alcoholic cosmetic formulations comprising the aqueous composition according to claim 1, in which the weight ratio between the solubilizing agent and the essential oil is comprised between 3:1 and 8:1.

4. Water-based cleansing formulations comprising the aqueous composition according to claim 1, in which the weight ratio between the solubilizing agent and the essential oil is comprised between 0.2:1 and 5:1.

5. Aqueous composition according to claim 1, further containing a salt formed from an inorganic or organic monovalent cation and an anion of an organic or inorganic acid.

6. Aqueous composition according to claim 5, wherein said salt is chosen from sodium chloride, sodium lactate and ammonium chloride.

7. Aqueous composition according to claim 6, wherein said salt is chosen between sodium chloride and sodium lactate and the weight ratio of solubilizer:salt:essential oil is comprised between 0.1:10:1 and 10:0.1:1.

8. Aqueous or aqueous-alcoholic cosmetic formulation comprising the composition of claim 7, wherein the weight ratio solubilizer:salt:essential oil is comprised between

9. Water-based cleansing formulation comprising the composition of claim 7, wherein the weight ratio solubilizer:salt:essential oil is comprised between 0.25:4:1 and 5:0.5:1.

10. Use of a N-acyl derivative of proline of formula (I) 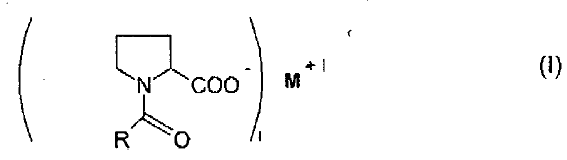 in which R is an alkyl radical from 5 to 21 carbon atoms and is obtained from fatty acids contained in coconut oil, M is chosen from sodium or potassium, I is 1, as solubilizing agent for essential oils.

11. Use according to claim 10 for the preparation of cosmetic formulations, wherein the weight ratio between the solubilizing agent and the essential oil is comprised between 3:1 and 8:1.

12. Use according to claim 10 for the preparation of water-based cleansing formulations, wherein the weight ratio between the solubilizing agent and the essential oil and/or fragrance is comprised between 0.2:1 and 5:1.

13. Use of the composition of claim 7 for the preparation of cosmetic formulations, wherein the weight ratio solubilizer:salt:essential oil is comprised between 1:10:1 and 6:0.1:1.

14. Use of the composition of claim 7 for the preparation of water-based cleansing formulations, wherein the weight ratio solubilizer:salt:essential oil is comprised between 0.25:4:1 and 5:0.5:1.

## Patentansprüche

1. Wässrige Zusammensetzung, enthaltend ein ätherisches Öl und ein Solubilisierungsmittel für das ätherische Öl, wobei das Solubilisierungsmittel zumindest ein N-Acyl-Derivat von Prolin der Formel (I) 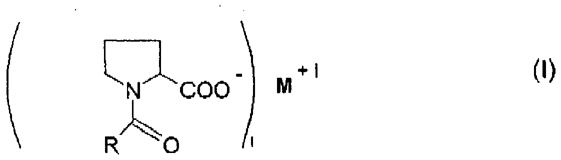 enthält, worin R ein Alkylradikal mit 5 bis 21 Kohlenstoffatomen ist und von in Kokosöl enthaltenen Fettsäuren gewonnen wird, M ausgewählt ist aus Natrium oder Kalium und I gleich 1 ist, wobei das Gewichtsverhältnis von dem Solubilisierungsmittel und dem ätherischen Öl zwischen 0,1:1 und 10:1 liegt.

2. Wässrige Zusammensetzung nach Anspruch 1, wobei das Solubilisierungsmittel aus einem Gemisch aus N-Acyl-Derivaten der Formel (I) mit verschiedenen gesättigten Alkylresten R mit 7 bis 20 Kohlenstoffatomen besteht.

3. Wässrige oder wässrig-alkoholische kosmetische Präparate, welche eine wässrige Zusammensetzung nach Anspruch 1 enthalten, wobei das Gewichtsverhältnis von dem Solubilisierungsmittel und dem ätherischen Öl zwischen 3:1 und 8:1 liegt.

4. Wasserbasierte Reinigungspräparate, welche die wässrige Zusammensetzung nach Anspruch 1 enthalten, wobei das Gewichtsverhältnis von dem Solubilisierungsmittel und dem ätherischen Öl zwischen 0,2:1 und 5:1 liegt.

5. Wässrige Zusammensetzung nach Anspruch 1, ferner enthaltend ein Salz, das aus einem anorganischen oder organischen, einwertigen Kation und einem Anion einer organischen oder anorganischen Säure gebildet ist.

6. Wässrige Zusammensetzung nach Anspruch 5, wobei das Salz ausgewählt ist aus Natriumchlorid, Natriumlactat und Ammoniumchlorid.

7. Wässrige Zusammensetzung nach Anspruch 6, wobei das Salz ausgewählt ist zwischen Natriumchlorid und Natriumlactat und das Gewichtsverhältnis von Solubilisierungsmittel, Salz und ätherischem Öl zwischen 0,1:10:1 und 10:0,1:1 liegt.

8. Wässriges oder wässrig-alkoholisches kosmetisches Präparat, welches die wässrige Zusammensetzung nach Anspruch 7 enthält, wobei das Gewichtsverhältnis von Solubilisierungsmittel, Salz und ätherischem Öl zwischen 1:10:1 und 6:0,1:1. liegt.

9. Wasserbasiertes Reinigungspräparat, welches die Zusammensetzung nach Anspruch 7 enthält, wobei das Gewichtsverhältnis von Solubilisierungsmittel, Salz und ätherischem Öl zwischen 0,25:4:1 und 5:0,5:1 liegt.

10. Verwendung eines N-Acyl-Derivats von Prolin der Formel (I) 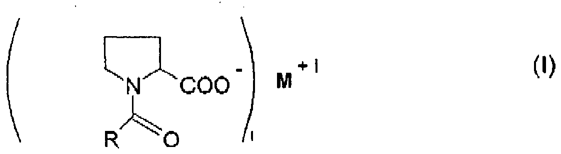 worin R ein Alkylradikal mit 5 bis 21 Kohlenstoffatomen ist und von in Kokosöl enthaltenen Fettsäuren gewonnen wird, M ausgewählt ist aus Natrium oder Kalium und I gleich 1 ist,
als Solubilisierungsmittel für ätherische Öle.

11. Verwendung nach Anspruch 10 für die Herstellung von kosmetischen Präparaten, wobei das Gewichtsverhältnis von dem Solubilisierungsmittel und dem ätherischen Öl zwischen 3:1 und 8:1 liegt.

12. Verwendung nach Anspruch 10 für die Herstellung von wasserbasierten Reinigungspräparaten, wobei das Gewichtsverhältnis von dem Solubilisierungsmittel und dem ätherischen Öl und/oder Duftstoff zwischen 0,2:1 und 5:1 liegt.

13. Verwendung der Zusammensetzung nach Anspruch 7 für die Herstellung von kosmetischen Präparaten, wobei das Gewichtsverhältnis von Solubilisierungsmittel, Salz und ätherischem OI zwischen 1:10:1 und 6:0,1:1 liegt.

14. Verwendung der Zusammensetzung nach Anspruch 7 für die Herstellung von wasserbasierten Reinigungspräparaten, wobei das Gewichtsverhältnis von Solubilisierungsmittel, Salz und ätherischem Öl zwischen 0,25:4:1 und 5:0,5:1 liegt.

## Revendications

1. Composition aqueuse contenant une huile essentielle et un agent solubilisant pour ladite huile essentielle, ledit agent solubilisant comprenant au moins un dérivé N-acyle de proline de formule (I) 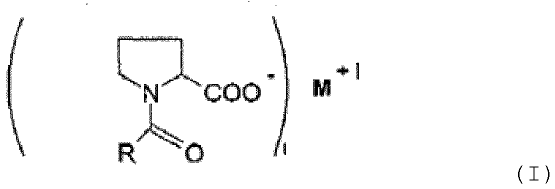 dans laquelle R est un radical alkyle de 5 à 21 atomes de carbone et est obtenu à partir d'acides gras contenus dans l'huile de noix de coco, M est choisi parmi le sodium ou le potassium, I est 1, le rapport en poids entre l'agent solubilisant et l'huile essentielle étant compris entre 0,1:1 et 10:1.

2. Composition aqueuse selon la revendication 1, dans laquelle l'agent solubilisant est constitué d'un mélange de dérivés N-acyle de formule (I) ayant différents résidus alkyle saturés R ayant de 7 à 20 atomes de carbone.

3. Formulations cosmétiques aqueuses ou hydro-alcooliques comprenant la composition aqueuse selon la revendication 1 dans lesquelles le rapport en poids entre l'agent solubilisant et l'huile essentielle est compris entre 3:1 et 8:1.

4. Formulations nettoyantes à base aqueuse comprenant la composition aqueuse selon la revendication 1 dans lesquelles le rapport en poids entre l'agent solubilisant et l'huile essentielle est compris entre 0,2:1 et 5:1.

5. Composition aqueuse selon la revendication 1, contenant en outre un sel formé d'un cation monovalent inorganique ou organique et un anion d'un acide organique ou inorganique.

6. Composition aqueuse selon la revendication 5, dans laquelle ledit sel est choisi parmi le chlorure de sodium, le lactate de sodium et le chlorure d'ammonium.

7. Composition aqueuse selon la revendication 6, dans laquelle ledit sel est choisi entre le chlorure de sodium et le lactate de sodium et le rapport en poids de solubilisant:sel:huile essentielle est compris entre 0,1:10:1 et 10:0,1:1.

8. Formulation cosmétique aqueuse ou hydro-alcoolique comprenant la composition de la revendication 7, dans laquelle le rapport en poids de solubilisant:sel:huile essentielle est compris entre 1:10:1 et 6:0,1:1.

9. Formulation nettoyante à base aqueuse comprenant la composition de la revendication 7, dans laquelle le rapport en poids de solubilisant:sel:huile essentielle est compris entre 0,25:4:1 et 5:0,5:1.

10. Utilisation d'un dérivé N-acyle de proline de formule (I) 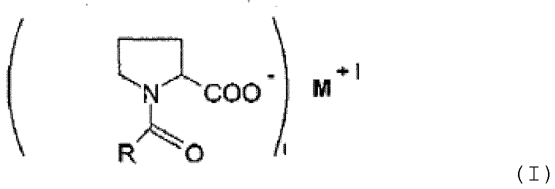 dans laquelle R est un radical alkyle de 5 à 21 atomes de carbone et est obtenu à partir d'acides gras contenus dans l'huile de noix de coco, M est choisi parmi le sodium ou le potassium, I est 1, en tant qu'agent solubilisant pour des huiles essentielles.

11. Utilisation selon la revendication 10 pour la préparation de formulations cosmétiques, dans lesquelles le rapport en poids entre l'agent solubilisant et l'huile essentielle est compris entre 3:1 et 8:1.

12. Utilisation selon la revendication 10 pour la préparation de formulations nettoyantes à base aqueuse, dans lesquelles le rapport en poids entre l'agent solubilisant et l'huile essentielle et/ou parfum est compris entre 0,2:1 et 5:1.

13. Utilisation de la composition de la revendication 7 pour la préparation de formulations cosmétiques, dans lesquelles le rapport en poids de solubilisant:sel:huile essentielle est compris entre 1:10:1 et 6:0,1:1.

14. Utilisation de la composition de la revendication 7 pour la préparation de formulations nettoyantes à base aqueuse, dans lesquelles le rapport en poids de solubilisant:sel:huile essentielle est compris entre 0,25:4:1 et 5:0,5:1.
